# EUROPEAN PATENT APPLICATION

(11) **EP 4 450 123 A1**
(43) Date of publication of application: **23.10.2024**
(21) Application number: 23169189.0
(22) Date of filing: 21.04.2023
(51) Int. Cl.: A61N 1/372, A61N 1/05, A61B 5/25, A61B 5/388

(54) **SYSTEMS AND METHODS FOR STIMULATION ARTIFACT REDUCTION IN NEURAL SENSING**

(71) Applicant: BIOTRONIK SE & Co. KG, 12359 Berlin (DE)
(72) Inventor: Baru, Marcelo, Tualatin, 97062 (US)
(74) Representative: Biotronik Corporate Services SE

(57) **Abstract**

The present invention relates to a device for neurostimulation, comprising: a pulse generator (100) configured to deliver a plurality of successive biphasic electrical stimulation phases (103), each comprising a stimulation pulse (103.a), an interphase period (103.b), and a charge balancing pulse (103.c), each pulse comprising an amplitude and a pulse width, at least a first electrode and a second electrode (102.a, 102.b) for delivering electrical biphasic stimulation phases (103), and wherein the device is configured to determine the slope and/or an absolute amplitude of a stimulation artifact generated by the respective biphasic electrical stimulation phase (103), wherein the device is further configured to reduce the amplitude and/or pulse width of the charge balancing pulse (103.c) of each biphasic electrical stimulation phase (103) with respect to the preceding biphasic electrical stimulation phase (103) until a final electrical stimulation phase (103) with reduced amplitude and/or pulse width of the charge balancing pulse (103.c) generates a stimulation artifact whose slope changed sign and/or having an absolute amplitude below a threshold, and wherein the device is configured to deliver therapy in form of at least one electrical biphasic stimulation phase having a charge balancing pulse comprising the amplitude and/or pulse width of the charge balancing pulse (103.c) of said final biphasic electrical stimulation phase (103), and wherein the device is configured to measure an evoked compound action potential (300) triggered by the delivered therapy.

## Description

The present invention relates to a device and method for providing neurostimulation, particularly spinal cord stimulation (SCS).

Such neurostimulation methods can be used for treatment of chronic pain. The so-called evoked compound action potential (ECAP) is a biopotential that represents neural activity triggered by neurostimulation such as SCS. Therefore, it is generally desirable to be able to sense ECAPs efficiently and with sufficient precision.

ECAP sensing typically utilizes stimulation pulses with active charge balancing given the fast-evoked-response travelling time to the sensing electrodes. Asymmetries in the electrochemical situations under each phase of the biphasic electrical stimulation phases, as well as electrode-tissue double-layer discharge during the interphase period, imply the standard approach of charge balancing with matched charge as the stimulation phase charge results in a residual voltage in tissue (overcompensating) that translates into a stimulation artifact (SA) for the sensing of the evoked response.

Thus, ECAP sensing is usually obscured by this SA as the latter is typically orders of magnitude larger. Hence, it is desirable to be able to employ a stimulation/sensing technique that minimizes the SA to reduce the sensing front-end requirements, in particular the input dynamic range. Furthermore, it is desirable to cancel out the remnant SA for clean evoked response sensing. Signal processing for this purpose, to remove the remnant SA, may involve averaging and curve fitting (e.g. exponential fitting).

Particularly, US Patent 10,183,168 discloses systems and methods where information about the charge injection process of the programmed stimulation phase is utilized to determine the adequate balancing phase that minimizes the electrode potential excursions without adding a compensatory phase or extra circuitry post biphasic stimulation. This minimizes the SA. However, such systems and methods are relatively computing intensive.

Furthermore, US Patent Application 2022/0142539 teaches to measure the ECAP response during a modified active charge balancing phase that is prolonged in time, and therefore of smaller amplitude, to have a smaller but constant SA.

Based on the above, the problem to be solved by the present invention is to provide a system and a method that utilize a stimulation scheme that minimizes the residual charge in tissue prior to ECAP sensing, when using different electrodes for stimulation and sensing. This is particularly suitable for closed-loop SCS control.

This problem is solved by a device having the features of claim 1 and a method having the features of claim 10. Preferred embodiments of these aspects of the present invention are stated in the corresponding dependent claims and are described below.

According to claim 1, a device for providing electrical stimulation is disclosed, the device comprising: a pulse generator configured to deliver a plurality of successive biphasic electrical stimulation phases, each comprising a stimulation pulse, a successive interphase period, and a successive charge balancing pulse, each pulse comprising an amplitude and a pulse width, and at least a first electrode and a second electrode for delivering the biphasic electrical stimulation phases, and wherein the pulse generator is configured to determine the slope and/or the absolute amplitude of an SA generated by the respective biphasic electrical stimulation phase, wherein the pulse generator is further configured to reduce the amplitude and/or pulse width of the charge balancing pulse of each biphasic electrical stimulation phase with respect to the preceding biphasic electrical stimulation phase until a final electrical stimulation phase with reduced amplitude and/or pulse width of the charge balancing pulse generates an SA whose slope changed sign (with respect to the previous biphasic electrical stimulation phase) and/or has an absolute amplitude below a threshold, and wherein the pulse generator is configured to deliver therapy in form of at least one electrical biphasic stimulation phase having a charge balancing pulse comprising the amplitude and/or pulse width of the charge balancing pulse used for said final biphasic electrical stimulation phase, and wherein the pulse generator is configured to measure an evoked compound action potential (ECAP) triggered by the delivered therapy.

Signal processing techniques for SA slope determination may involve averaging and curve fitting (e.g. exponential fitting) considering any minimum remnant SA will still be an order of magnitude larger than the ECAP signal to be sensed.

In other words, the present invention particularly proposes adjusting the charge injected in the balancing pulse, by reducing the balancing current amplitude and/or pulse width, utilizing test biphasic phases identical to the ones for therapy, sensing the SA, and detecting when the SA slope changes sign and/or the absolute amplitude of the SA is at a minimum as that is indicative the charge balancing phase has returned the electrode potentials close to their pre-pulsing values resulting in minimal charge left in tissue.

The electrode-tissue double layer voltage is understood as the voltage developed across the electrode-tissue interface. Such voltage cannot be measured directly in a chronic implant as that would require a long-term non-polarizable electrode reference (e.g. of silver/silver chloride Ag/AgCl) to be implanted. Hence the present invention indirectly senses the remnant charge in these double layers, and thus the SA, via the sensing electrodes voltage difference

Preferably, sensing of the evoked response occurs after the adjusted biphasic phase terminates. Once sensing is completed, any remnant charge on the DC-blocking capacitors (preferably used for safety in the pulse generator) is shared with tissue via a passive charge balancing phase (e.g. electrodes are connected to system ground). The lossy nature of the electrode-tissue double-layer interfaces results in such transferred charge being dissipated within the time for the worst case of maximum impedance and stimulation frequency in traditional SCS tonic therapy without associated side effects (i.e. undesired stimulation).

By reducing the required active balancing phase charge that needs to be delivered, the system and method of the present invention also have the benefit of reducing the power consumption associated with closed-loop stimulation compared with prior art.

Particularly, according to an embodiment of the present invention, unlike prior art, the ECAP response is not sensed during the active charge balancing phase.

In a preferred embodiment, the pulse generator is an implantable pulse generator. Furthermore, in a preferred embodiment, the system is configured to provide closed-loop spinal cord stimulation (SCS).

Further, according to a preferred embodiment of the present invention, the system comprises a plurality of electrodes for delivering the electrical biphasic stimulation phases that are assembled on implantable leads such as percutaneous or paddle leads.

Furthermore, according to a preferred embodiment of the present invention, each electrode is connected in series to a DC blocking capacitor present in the pulse generator for the purpose of delivering therapy.

Furthermore, in a preferred embodiment of the present invention, the pulse generator is configured to deliver the respective biphasic electrical stimulation phases via at least a first electrode and a second electrode of said plurality of electrodes. Preferably, in an embodiment, the pulse generator is configured to sense the stimulation artifact (SA) using at least a third electrode and a fourth electrode being spaced apart from said first and second electrodes via which the respective biphasic electrical stimulation phase is delivered. Furthermore, in a preferred embodiment, the pulse generator comprises a front-end circuit connected to said two electrodes for sensing the SA.

According to yet another preferred embodiment of the present invention, the pulse generator is configured to discharge the DC blocking capacitors in tissue of a human or animal patient after having sensed the evoked compound action potential (ECAP) and before delivering the next biphasic electrical stimulation phase.

Particularly, according to a preferred embodiment of the present invention, the device is configured to measure the voltage difference between at least two electrodes after delivery of the biphasic electrical stimulation phases which comprises the SA. The pulse generator is configured to determine the slopes of the different SAs and to find that the absolute amplitude of the SA is below said threshold, in case the absolute slopes are equal within a pre-defined tolerance after changing signs.

According to a further embodiment of the present invention, for keeping the SA at a minimum, the device is configured to repeat delivering a plurality of biphasic electrical stimulation phases (e.g. periodically). Here, in turn, each biphasic electrical stimulation phase comprises a stimulation pulse, a successive interphase period, and a successive charge balancing pulse, each pulse comprising an amplitude and a pulse width, wherein the device is configured to determine the slope and/or the absolute amplitude of the SA generated by the respective biphasic electrical stimulation phases and to reduce the amplitude and/or pulse width of the charge balancing pulse of each succeeding biphasic electrical stimulation phase with respect to the preceding biphasic electrical stimulation phase until a final biphasic electrical stimulation phase with reduced amplitude and/or pulse width of the charge balancing pulse generates an SA whose slope changed sign and/or has an absolute amplitude below the threshold.

The device is further configured to deliver therapy in form of at least one electrical biphasic stimulation phase having a charge balancing pulse comprising the amplitude and/or pulse width of the charge balancing pulse used for said final biphasic electrical stimulation phase of the repeated delivery of the plurality of biphasic electrical stimulation phase, and wherein the pulse generator is configured to measure an evoked compound action potential (ECAP) triggered by the delivered further therapy. Thus, the invention allows to repeatedly check if the SA still has the required minimal absolute amplitude that allows efficient ECAP sensing.

According to yet another aspect of the present invention, a method for neurostimulation is disclosed, wherein the method preferably uses a device according to the present invention, and wherein the method comprises the steps of:
a) Delivering, via at least a first and a second electrode, a biphasic electrical stimulation phase comprising a stimulation pulse, a successive interphase period, and a successive charge balancing pulse, each pulse comprising an amplitude and a pulse width,
b) automatically determining the slope and/or absolute amplitude of a stimulation artifact (SA) generated by the biphasic electrical stimulation phase, and automatically reducing the amplitude and/or pulse width of the charge balancing pulse of the biphasic electrical stimulation phase,
c) repeating steps a) and b) as long as the SA slope does not change sign and/or the absolute amplitude is above or equal to a threshold, wherein the reduced amplitude and/or pulse width of the charge balancing pulse of step b) is used in step a), otherwise
d) delivering therapy in form of at least one electrical biphasic stimulation phase having a charge balancing phase comprising the reduced amplitude and/or pulse width of the charge balancing pulse of step b), and
e) automatically measuring an evoked compound action potential (ECAP) triggered by the delivered therapy.

According to an aspect, the inventive method further comprises the step of:
- sensing the stimulation artifact (SA) using a third electrode and a fourth electrode being spaced apart from said first electrode and second electrode via which the respective biphasic electrical stimulation phase is delivered.

Furthermore, according to an embodiment, the inventive method further comprises the step of:
- discharging DC blocking capacitors after having measured the ECAP, wherein each electrode is connected in series to a DC blocking capacitor.

According to an aspect of the invention, the proposed method further comprises the steps of:
- measuring the voltage difference between the third and the fourth electrode after delivery of the biphasic electrical stimulation phases, i.e. sensing the SA,
- determining the slopes of SAs,
- detecting that the absolute amplitude of the SA is below said threshold, in case the absolute SA slopes are equal within a pre-defined tolerance after changing sign.

The method of the present invention can be further specified by the features and embodiments described in conjunction with the device according to the present invention.

In the following, preferred embodiments as well as further features and advantages are described with reference to the Figures, wherein
- Fig. 1: shows a simulation of a biphasic stimulation current phase circulating between two electrodes (i.e. bipolar stimulation) when the electrode-tissue impedance model is the one developed by Scott and Single,
- Fig. 2: shows a configuration for estimating the return of the electrode potentials back to their pre-pulsing values via injecting biphasic stimulation current phases in the therapy electrodes and sensing the reduction in SA slope and polarity change, and minimum absolute amplitude in the case of equal slopes,
- Fig. 3: shows that once the charge balancing phase 103.c is defined, closed-loop stimulation is delivered, wherein ECAP signal 300 is sensed via front-end circuit 200 after the adjusted biphasic stimulation current phase 103 has been delivered,
- Fig. 4: shows the simulated developed voltage (0.5 s from stimulation start to observe a more steady-state stimulation) between the sensing electrodes 102.c, 102.d of Fig. 2,
- Fig. 5: shows for example the discharge of the DC-blocking capacitors voltage 500, 501 associated with biphasic stimulation current phase 103 into tissue, and the double layer voltages 104, 105, during period 301 of Fig. 3, for the case of maximum expected impedance in SCS (i.e. 4 kS2), and at maximum stimulation frequency of traditional tonic therapy (i.e. 130 Hz)

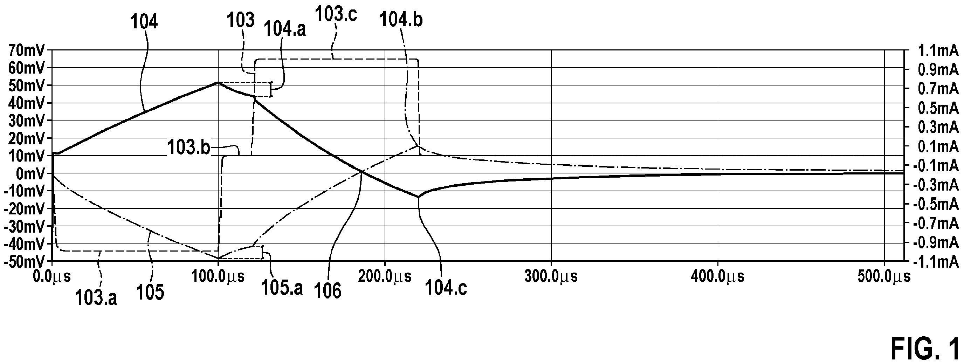

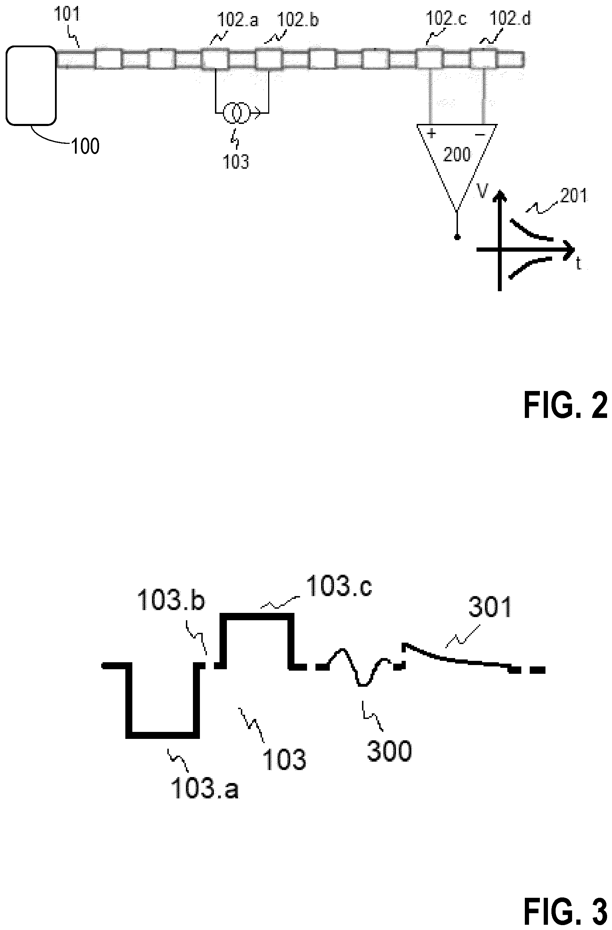

Fig. 2 schematically shows, in conjunction with Fig. 1, a device for neurostimulation according to the present invention, comprising a pulse generator 100 configured to deliver a plurality of successive biphasic electrical stimulation phases, each comprising a stimulation pulse 103.a, an interphase period 103.b, and a charge balancing phase 103.c, each pulse comprising an amplitude and a pulse width, and wherein the system is configured to determine the slope and/or the absolute amplitude of a stimulation artifact (SA) (the difference of voltages 104.b, 104.c at the end of the biphasic pulse in Fig. 1) generated by the respective biphasic electrical stimulation phases, wherein the system is further configured to reduce the amplitude and/or pulse width of the charge balancing phase 103.c of each biphasic electrical stimulation phase with respect to the preceding biphasic electrical stimulation phase until a final biphasic electrical stimulation phase with reduced amplitude and/or pulse width of the charge balancing pulse generates an SA having an absolute amplitude below a threshold, and wherein the device is configured to deliver therapy in form of at least one electrical biphasic stimulation pulse having a charge balancing phase comprising the amplitude and/or pulse width of the charge balancing phase of said final biphasic electrical stimulation pulse, and wherein the device is configured to measure an evoked compound action potential (ECAP) triggered by the delivered therapy.

According to a preferred embodiment, the device according to the present invention comprises an implantable pulse generator (IPG) 100 connected to one or more percutaneous or paddle leads 101 with multiple electrodes 102. Electrical stimulation is preferably delivered via series DC-blocking capacitors with each electrode 102 (present in the IPG) for single-fault safety. Different front-end configurations for evoked compound action potential (ECAP) sensing have been previously described in US Patent 10,183,168.

Fig. 1 shows an LTSpice^{®} simulation of a biphasic stimulation current phase 103 circulating between two electrodes 102 (i.e. bipolar stimulation) when the electrode-tissue impedance model is the one developed by Scott and Single (J. Scott and P. Single, "Compact Nonlinear Model of an Implantable Electrode Array for Spinal Cord Stimulation (SCS)," in IEEE Transactions on Biomedical Circuits and Systems, vol. 8, no. 3, pp. 382-390, June 2014, doi: 10.1109/TBCAS.2013.2270179). In this example, the biphasic stimulation current phase 103 consists of a stimulation pulse 103.a (e.g., 1 mA, 0.1 ms), an interphase period 103.b (e.g., 20 µs), and a charge balancing pulse 103.c (e.g., 1 mA, 0.1 ms). Voltage profiles 104 and 105 are the electrode-tissue double layer voltages of the anode and cathode respectively. As it can be seen, during the interphase period 103.b, these double layers re-distribute charge resulting in voltage changes 104.a and 105.a respectively. Basically, chemical reactions at the double layer interfaces proceed with some probability. After the injection of the stimulation charge of pulse 103.a, the rate of less favorable reactions increases. The matched charge balancing phase 103.c (having the same charge as 103.a) thus overcompensates resulting in voltages 104.b, 104.c at the end of the biphasic phase. This remaining charge creates the stimulation artifact (SA).

In a preferred embodiment, the crossing 106 of the double-layer voltages 104, 105, which happens somewhere before the end of the charge balancing pulse 103.c, is detected by injecting biphasic stimulation phases 103 in the therapy electrodes, for example in electrodes 102.a, 102.b, and sensing, e.g. via electrodes 102.c, 102.d voltage difference and front-end 200, the reduction in SA slope and polarity change 201, and minimum SA amplitude in the case of equal slope, as shown in Figure 2.

Preferably, the electrodes 102.c, 102.d are a couple of electrodes away from stimulation electrodes 102.a, 102.b to permit the stimulation biphasic phase to complete before enabling sensing. To search for the change in SA slope, successive biphasic stimulation phases 103 either reduce the charge balancing pulse 103.c amplitude or pule width in the steps specified in the IPG 100 (e.g., 50 µA and 3.8 µs). Particularly, for short interphase periods 103.b, it is preferred to reduce the amplitude of the charge balancing pulse 103.c as this reduces the stimulation threshold for the stimulation pulse 103.a. However, finer percentage reduction in charge may be achieved via reducing the pulse width of the charge balancing pulse 103.c instead.

Once the charge balancing pulse 103.c is defined, closed-loop stimulation is preferably delivered as illustrated in Fig. 3. The ECAP signal 300 is sensed via front-end circuit 200 after the adjusted biphasic phase 103 is delivered. Signal processing to remove any residual SA may involve averaging and curve fitting (e.g. exponential fitting).

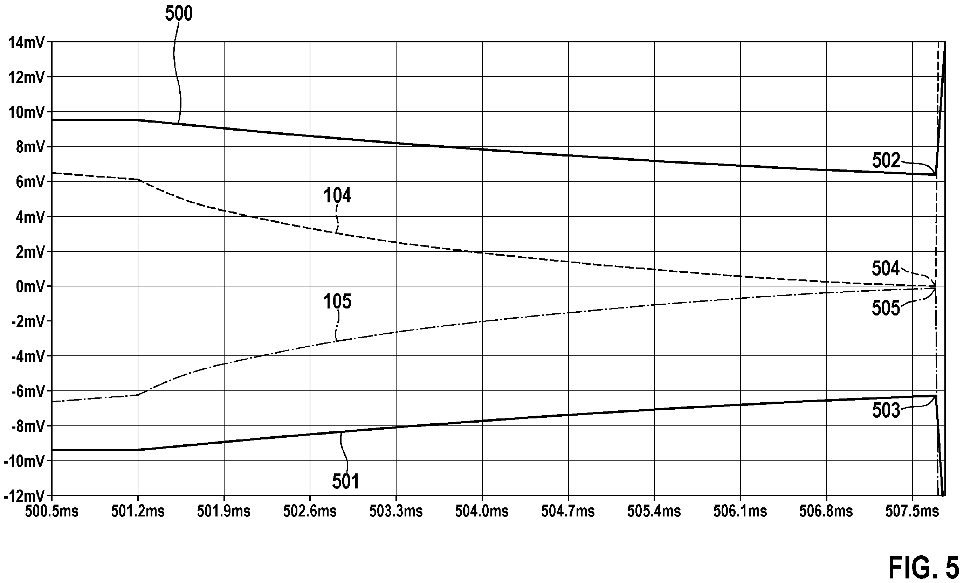

In the case of a percutaneous SCS lead 101 for example, where each electrode 102 particularly has a nominal length of 4 mm and the inter-electrode spacing is particularly 3 mm, the ECAP signal 300 corresponding to the fastest conducting nerve fibers (travelling at 55 m/s) will appear at around 300 µs from the start of the stimulation pulse 103.a in the embodiment of Fig. 2. Following sensing, the DC-blocking capacitors associated with each participating therapy electrode 102 are preferably discharged through tissue during period 301. This will prevent voltage runaway in the DC-blocking capacitors for continuous stimulation delivery. The transferred charge to tissue will be dissipated with a time constant as described later on (see Fig. 5).

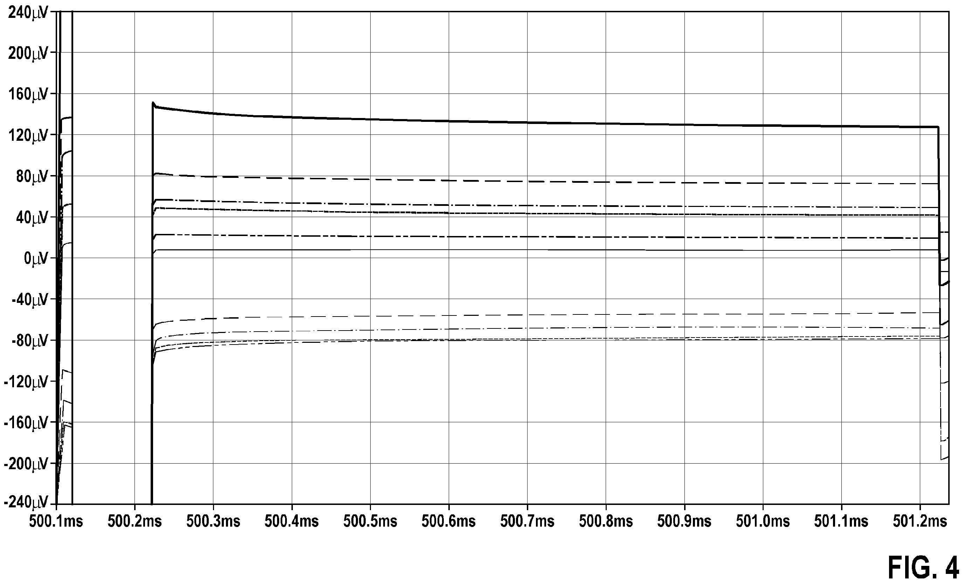

Fig. 4 shows the simulated developed voltage (0.5 s from stimulation start to observe a more steady-state stimulation) between the sensing electrodes 102.c, 102.d of Fig. 2, starting at 300 µs from the start of the stimulation pulse 103.a, in the case of varying charge balancing pulse 103.c amplitude. In this example, the minimum slope is achieved when the charge balancing pulse 103.c amplitude equals 0.75 mA (400, from 500.3 ms onwards). That implies the charge balancing pulse 103.c only requires 75% of the stimulation pulse 103.a charge for SA minimization. The simulation sweep varied the charge balancing pulse 103.c amplitude between 0.55 mA and 1.00 mA in steps of 0.05 mA. The SA will not be fully eliminated but it will almost be constant given the minimum slope thus simplifying ECAP sensing via back-end signal processing.

Furthermore, Fig. 5 illustrates for example the discharge of the DC-blocking capacitors 500, 501 associated with biphasic stimulation phase 103 into tissue, and the double layer voltages 104, 105, during period 301 of Fig. 3, for the case of maximum expected impedance in SCS (i.e. 4 kΩ), and at maximum stimulation frequency of traditional tonic therapy (i.e. 130 Hz). As it can be seen, in this particular case of a biphasic phase 103 of 1 mA, 0.1 ms, the remnant accumulated voltages 502, 503 in the DC-blocking capacitors, before the next stimulation pulse 103.a, are only a few mV. Also, the two double layers have relaxed to voltages 504, 505 of hundreds of µV. For biphasic pulses 103 of higher charge, more millivolts may be accumulated in the DC-blocking capacitors, but stimulation will still be delivered with negligible power consumption impact. Actually, with the proposed stimulation scheme herein disclosed, power consumption is reduced compared with state-of-the-art stimulation for closed-loop neurostimulation, as the active charge balancing pulse is typically less than the stimulation pulse.

The search for the optimum charge balancing pulse 103.c amplitude or pulse width (to minimize the SA) preferably takes place every time stimulation is re-programmed or the patient changes body positions. To assist the detection of body change positions, in a preferred embodiment, IPG 100 includes an accelerometer. Alternatively, the search for the optimum is performed periodically in time.

The present invention advantageously achieves a simple indirect approach to minimize the SA for ECAP sensing, in particular for closed-loop SCS, by splitting the charge balancing scheme. Furthermore, power consumption is advantageously reduced compared with other closed-loop stimulation approaches for ECAP sensing as the charge balancing pulse is typically delivered with less charge than the stimulation pulse.

## Claims

1. A device for neurostimulation, comprising:
a pulse generator (100) configured to deliver a plurality of successive biphasic electrical stimulation phases (103), each comprising a stimulation pulse (103.a), an interphase period (103.b), and a charge balancing pulse (103.c), each pulse comprising an amplitude and a pulse width,
at least a first electrode and a second electrode (102.a, 102.b) for delivering electrical biphasic stimulation phases (103),
and wherein the device is configured to determine a slope and/or an absolute amplitude of a stimulation artifact generated by the respective biphasic electrical stimulation phase (103), wherein the pulse generator (100) is further configured to reduce the amplitude and/or pulse width of the charge balancing pulse (103.c) of each biphasic electrical stimulation phase (103) with respect to the preceding biphasic electrical stimulation phase (103)
until a final electrical stimulation phase (103) with reduced amplitude and/or pulse width of the charge balancing pulse (103.c) generates a stimulation artifact whose slope changed sign and/or has an absolute amplitude below a threshold,
and wherein the device is configured to deliver therapy in form of at least one electrical biphasic stimulation phase (103) having a charge balancing pulse (103.c) comprising the amplitude and/or pulse width of the charge balancing pulse (103.c) of said final biphasic electrical stimulation phase (103),
and wherein the device is configured to measure an evoked compound action potential (300) triggered by the delivered therapy.

2. The device according to claim 1, wherein each of the at least first electrode and the second electrode (102.a, 102.b) is connected in series to at least one DC blocking capacitor in the pulse generator (100).

3. The device according to claim 1 or 2, wherein the pulse generator (100) is configured to deliver the respective biphasic electrical stimulation phase (103) via a first electrode and a second electrode (102.a, 102.b) of said plurality of electrodes (102).

4. The device according to claim 3, wherein the device is configured to sense the stimulation artifact using a third electrode and a fourth electrode (102.c, 102.d) being spaced apart from first electrode and second electrode (102.a, 102.b) via which the respective biphasic electrical stimulation phase (103) is delivered.

5. The device according to claim 4, wherein the device comprises a front-end circuit (200) connected to said third electrode and fourth electrode (102.c, 102.d) for sensing the stimulation artifact.

6. The device according to claim 2 or according to one of the claims 3 to 5 insofar referring to claim 2, wherein the device is configured to discharge (301) the DC blocking capacitors after having measured the evoked compound action potential (300).

7. The device according to one of the preceding claims, wherein for keeping the stimulation artifact at a minimum, the device is configured to repeat delivering a plurality of biphasic electrical stimulation phases (103), each comprising a stimulation pulse (103.a), an interphase period (103.b), and a charge balancing pulse (103.c) comprising an amplitude and a pulse width, wherein the device is configured to determine the slope and/or the absolute amplitude of a stimulation artifact generated by the respective biphasic electrical stimulation phase (103) and to reduce the amplitude and/or pulse width of the charge balancing pulse (103.c) of each succeeding biphasic electrical stimulation phase (103) with respect to the preceding biphasic electrical stimulation phase (103) until a final biphasic electrical stimulation phase (103) with reduced amplitude and/or pulse width of the charge balancing pulse (103.c) generates a stimulation artifact whose slope changed sign and/or has an absolute amplitude below the threshold.

8. A method for neurostimulation, particularly using a device according to one of the preceding claims, the method comprising the steps of:
a) delivering, via at least a first and a second electrode (102.a, 102.b), a biphasic electrical stimulation phase (103) comprising a stimulation pulse (103.a), an interphase period (103.b), and a charge balancing pulse (103.c), each pulse comprising an amplitude and a pulse width,
b) determining the absolute amplitude of a stimulation artifact generated by the biphasic electrical stimulation phase (103), and reducing the amplitude and/or pulse width of the charge balancing pulse (103.c) of the biphasic electrical stimulation phase (103),
c) repeat steps a) and b) if
the absolute amplitude of the stimulation artifact is above or equal to a threshold, or if the slope of the stimulation artifact has not changed sign,
wherein the reduced amplitude and/or pulse width of the charge balancing pulse (103.c) of step b) is used in step a), otherwise
d) delivering therapy in form of at least one electrical biphasic stimulation phase (103) having a charge balancing pulse (103.c) comprising the reduced amplitude and/or pulse width of the charge balancing pulse (103.c) of step b), and
e) measuring an evoked compound action potential (300) triggered by the delivered therapy.

9. The method according to claim 8, further comprising the step of:
- sensing the stimulation artifact using a third electrode and a fourth electrode (102.c, 102.d) being spaced apart from said first electrode and second electrode (102.a, 102.b) via which the respective biphasic electrical stimulation phase (103) is delivered.

10. The method according to claim 8 or 9, further comprising the step of:
- discharging DC blocking capacitors after having measured the evoked compound action potential (300), wherein each electrode (102.a, 102.b) is connected in series to at leasr one DC blocking capacitor.

11. The method according to claim 9 or 10, further comprising the steps of:
- measuring the voltage difference between the third and the fourth electrode after delivery of the biphasic electrical stimulation phases (103),
- determining the slopes of the SAs,
- detecting that the absolute amplitude of the stimulation artifact is below said threshold, in case the SA slopes are equal within a pre-defined tolerance.
